# EUROPEAN PATENT SPECIFICATION

(11) **EP 3 583 963 B1**
(45) Date of publication and mention of the grant of the patent: **06.04.2022**
(21) Application number: 18178231.9
(22) Date of filing: 18.06.2018
(51) Int. Cl.: A61M 1/16, A61M 1/36

(54) **DEVICE AND METHOD FOR TREATMENT AND/OR PROPHYLAXIS OF DECOMPRESSION ILLNESS**
VORRICHTUNG UND VERFAHREN ZUR BEHANDLUNG UND/ODER PROPHYLAXE VON DEKOMPRESSIONSKRANKHEIT
DISPOSITIF ET PROCÉDÉ POUR LE TRAITEMENT ET/OU LA PROPHYLAXIE D'UNE MALADIE DE DÉCOMPRESSION

(43) Date of publication of application: 25.12.2019
(73) Proprietor: OkeanosDeco UG, 81243 München (DE)
(72) Inventor: Tillmanns, Sascha, 81243 München (DE)
(74) Representative: Schiweck Weinzierl Koch Patentanwälte Partnerschaft mbB

(56) References cited:
- US-A1- 2007 239 097
- US-A1- 2010 143 192
- US-A1- 2017 326 282
- US-B1- 6 267 926

## Description

The present application relates to a device for treatment and/or prophylaxis of decompression illness, in particular for treatment and/or prophylaxis of decompression sickness and arterial gas embolism (AGE), both being caused by expansion of respiratory gases during ascent from a dive.

United States patent US 6 267 926 B1 discloses a device for removing entrained gases from liquids. United States patent application publication US 2010/143192 A1 discloses a method and a device for combined detection of bubbles and flow rate in a system for enriching a bodily fluid with a gas. United States patent application publication US 2007/239097 A1 discloses a bubble trap.

During scuba diving, a diver is provided with a mixture of gases, for example air, containing an appropriate amount of oxygen, i.e. in the physiological range of approximately 21 vol%. In order to be able to inflate the lungs against the pressure of the water column above the diver, the mixture of gasses is provided at a pressure corresponding to the hydrostatic pressure acting upon the body of the diver, in particular the thorax. With increasing depth of the dive the gas pressure is increased and in proportion thereto the quantity of gases dissolved in the blood and body tissues increases. Due to the fact that oxygen is metabolized while the other gasses of the gas mixture are not (e.g. nitrogen, helium), the latter will dissolve from the solution and body tissues when the hydrostatic pressure which the diver is exposed to is reduced during the decompression phase which is caused by the diver's ascent back to the surface.

It is well-known in the field of scuba diving that those ascents have to take place at a predefined, slow rate in order to prevent complications caused by too fast decompression. When a diver ascends too fast, the gasses dissolved in his blood and body tissues which are not metabolized may be released at a too high rate and form bubbles. Bubbles, in turn, can block peripheral capillaries, cause gas embolisms in the blood vessels of vital organs and lead to necroses of the surrounding tissues. Decompression sickness manifests itself in the accumulation of gas bubbles in the joints. If untreated, decompression sickness may lead to neurological disorders caused by functional losses in the central nervous system and even to death.

Most complications of the aforementioned type can be prevented or at least mitigated by following an ascent schedule which takes into account the depth of the dive and the duration of stay at a given depth. Based on the knowledge of the physiological and physical effects of scuba diving on the human body ascent tables have been established. As a further measure aimed at reducing the detrimental effects of scuba diving, the gas mixture provided to the diver may be optimized by replacing nitrogen with helium which has a lower solubility in body tissues. The use of helium further aims at preventing narcosis. Helium diffuses faster into and out of tissue than nitrogen. Depending on the duration and depths reached during a dive, this effect may lead to more or less stringent decompression duties after the dive. However, situations may occur in which rapid assents are still required, especially in the case of an accident during a dive. Even the use of an optimized breathing atmosphere cannot prevent severe physiological consequences from too rapid ascents.

The effects of decompression illness following a too rapid ascent can be mitigated to a high degree or, in the optimal case, almost prevented completely, if the affected diver is brought into a decompression chamber as fast as possible. In the decompression chamber, the diver is initially exposed to a higher pressure, which corresponds to the hydrostatic pressure at a higher depth, and starting therefrom a slow ascent can be simulated. Unfortunately, decompression chambers are almost exclusively found on land and since they are expensive, even there they are not very commonplace. This means that a diver in need of treatment has to be transported to a decompression chamber first which results in an unwanted delay in treatment and ultimately increases the risk of suffering from the symptoms of decompression illness.

The present invention is limited by the set of claims and has been devised in view of the above mentioned problems and seeks to provide a compact device which may be kept at the site of diving, thus drastically shortening the crucial time until a diver in need of treatment is treated in order to counteract effects of decompression illness. In addition, the present invention may be used in combination with a decompression chamber to improve the treatment aimed at preventing detrimental effects related to decompression illness.

In various embodiments, a device for treatment and/or prophylaxis of decompression illness in a subject is provided, wherein both the effects of decompression illness and arterial gas embolism may be treated. The device comprises an input port which is configured to provide venous, arterial or peritoneal access to a subject to extract blood from the subject; a blood processing stage coupled to the input port which is configured to process the blood extracted from the subject; and an output port which is configured to provide venous, arterial or peritoneal access to infuse the processed blood back into the subject. The blood processing stage includes at least one pump to promote a flow of the blood through the blood processing stage; at least one bubble filter configured to remove gas bubbles from the extracted blood; a depressurizing module in which the blood flowing through the blood processing stage is depressurized to provoke formation of gas bubbles in the blood and their subsequent release from the blood, wherein the depressurizing module is a further module; and a gas exchange module comprising a gas reservoir and configured to provide gas exchange between the blood flowing through the blood processing stage and a gas reservoir.

The present device is configured to remove gasses which are present in the blood of the subject, e.g. physically dissolved or present in form of gas bubbles in the blood, in particular to provide a treatment for decompression illness. The device may be used to treat decompression illness or for prophylaxis before a scuba dive in order to preemptively lower the content of at least one gas or gas component which is present in the blood of the subject. For that purpose, blood which is obtained from the vein, artery or peritoneum of a subject is transported from one module to the next within the blood processing stage and is processed at the various modules in order to reduce the content of at least one gas in the blood of the subject. The at least one gas to be removed from the blood may be physically dissolved in the blood, i.e. its molecules may be more or less homogenously mixed with the blood. In addition, especially when the content of the at least one gas exceeds its solubility limit, the gas to be removed from the blood may coalesce and form gas bubbles within the blood. Most prominently, the at least one gas may comprise nitrogen.

The blood processing stage is coupled between the input port and the output port and comprises the modules (bubble filter, depressurizing modules, etc.) which are connected to one another via conduits/tubes. The device, in particular the blood processing stage, is a closed system in that the modules are connected to one another in an airtight/sealed manner. In particular, the blood drawn from a subject is not exposed to the surrounding atmosphere while being processed and may be infused back into the subject without being contaminated in any way. The input port and the output port may each include a cannula.

The at least one pump provided in the blood processing stage may be a peristaltic pump, for example. The pump is provided in order to promote a flow of the blood through the blood processing stage at a high enough rate (which is increased in comparison to the physiologic blood flow) in order to enable a high gas exchange rate in the gas exchange module. In particular, by means of the pump the blood may be pumped through the device at a pressure which is different from the physiologic blood pressure. Additionally, the at least one pump may help overcoming the underpressure in the depressurizing module.

The at least one bubble filter is configured to remove gas bubbles from the extracted blood. According to one exemplary embodiment, the bubble filter may include a container comprising an input and an output. The input may be provided at a wall of the container and the output may be provided at the bottom of the container. Blood flowing into the container will flow towards the bottom of the container, while bubbles contained in the blood tend to rise upwards, i.e. towards the top of the container, thus being separated from the blood. An opening may be provided at the top of the container through which the gas separated from the blood may be sampled and analyzed. The blood agglomerating at the bottom of the container is substantially free of gas bubbles and may be withdrawn from the container and transported to the next module.

The depressurizing module is a further module which is provided in order to remove gas from the blood. However, in distinction from the bubble filter, the depressurizing module is configured to remove at least one gas or gas component from the blood which is primarily dissolved in the blood, i.e. not forming macroscopic bubbles but primarily homogenously dissolved therein as single molecules or small agglomerations of molecules. According to one exemplary embodiment, the depressurizing module may comprise a chamber which has an input and an output. The blood flowing through the depressurizing module may be exposed to a pressure which is lower than the pressure in the part of the blood processing stage preceding the depressurizing module and/or lower than the pressure in the part of the blood processing stage being arranged behind the depressurizing module. The input and/or the output of the depressurizing module may comprise a check valve. Inside the depressurizing module, the pressure may be lowered to a pressure approximately 100 mbar to 500 mbar, more preferably approximately 200 mbar to 300 mbar less than the pressure of the blood in the portion of the blood processing stage excluding the depressurizing module. In other words, the blood flowing through the blood processing stage may be exposed to a pressure difference of 300 mbar or less between the input of the depressurizing module and its interior. A further pump, such as a peristaltic pump, may be arranged downstream of the depressurizing module in order to promote further transport of the blood from the depressurizing module against its relative underpressure (i.e. the lower pressure within the depressurizing module as compared to the pressure in the section of the blood processing stage before and behind the depressurizing module).

The gas exchange module comprises a gas reservoir in which a gas or gas mixture may be provided. In the gas exchange module, at least one gas is extracted from the blood and another gas is deposited in the blood from the gas reservoir in replacement of the withdrawn gas. In the gas exchange module, a gas exchange between the blood flowing through the gas exchange module and a gas reservoir takes place. Therefore, the gas exchange module may comprise two compartments, each compartment having an input and an output. The processed blood flows through one compartment (blood compartment) and at least one gas is provided in the other compartment of the gas exchange module, i.e. the gas reservoir. Both compartments share at least one outer wall. In accordance with a further embodiment, the gas exchange module may comprise a gas permeable membrane or film which is arranged between the gas reservoir and the blood compartment, i.e. such that its one side is in contact with the blood flowing through the blood compartment of the gas exchange module and its other side is facing the gas reservoir. In other words, the gas permeable membrane may be seen as interface between the blood in the blood compartment and the gas mixture provided in the gas reservoir which allows exchange of gasses between those two compartments. The membrane may have a predefined pore size which may be adapted to the gas molecules to be exchanged. In general, membranes known from oxygenators may be used for the purpose of the present invention. Such membranes are permeable for gas molecules, such as O2 and CO2 (which have dimensions in the sub-nanometer range), but are impermeable for red blood cells, which have a diameter in the range of 6-8 µm. According to various embodiments, the wall thickness of the membrane may be in the order of a few tens of micrometers, for example 50 µm, and the pore size of the membrane may be in the sub-micrometer range, for example in the order of 1 µm, for example 0.2 µm or less. A commercially available membrane suitable for the purpose of the present invention is the OXYPLUS^{®} membrane manufactured by 3M.

The pressure on both sides of the membrane may be substantially the same. In alternative embodiments, the pressure in the gas reservoir may be slightly higher than the pressure in the blood reservoir. In the gas exchange module, the gasses dissolved in the blood are made to follow their chemical potential gradient and migrate from the blood compartment across the membrane into the gas reservoir. In particular, the at least one target gas, i.e. the at least one gas which is to be removed from the blood, is substantially not present or present only at a very low concentration in the gas reservoir. Due to the strong difference in concentrations of the target gas in the blood reservoir and the gas reservoir, the target gas is forced to migrate from the blood in the blood compartment into the gas reservoir. In exchange, a replacement gas is drawn from the gas reservoir into the blood in the blood reservoir through the membrane. The replacement gas may be practically not present in the blood or it may be present in the blood only at a very low concentration. In any case, the concentration of the replacement gas in the gas reservoir is much higher than the concentration of that same gas in the blood, such that the replacement gas diffuses through the membrane in accordance with the chemical potential gradient. The target gas may include nitrogen and/or carbon dioxide and/or other inert gasses. The replacement gas may comprise oxygen and at least one further gas. In particular, the replacement gas may comprise oxygen and air or oxygen and an inert gas, such as helium. The output of the gas reservoir may be used to controllably release a portion of the gas mixture which is present in the gas reservoir, in particular to control the pressure within the gas reservoir.

The present device may be a portable device, e.g. including a battery for self-sufficient operation. The internal blood volume of the device, i.e. the blood volume which can be accommodated within the device between its input and output may be approximately in the range of 1 l to 500 ml.

In accordance with a further embodiment, the gas exchange module may comprise a gas supply comprising oxygen and preferably at least one inert gas, such as helium. Therefore, oxygen and preferably the at least one inert gas may be supplied to the gas reservoir and act as replacement gasses. Each of the gasses may be provided independently such that a predefined mixture of gasses may be provided in the gas reservoir. For example, the gas reservoir may be supplied with 100% oxygen. Alternatively, the gas reservoir may be provided with a mixture of oxygen and at least one further gas component, e.g. an inert gas or air (air already being a gas mixture itself).

In accordance with a further embodiment of the device, the blood processing stage may further include a controller which is configured to control the gas flow between the gas supply and the gas reservoir of the gas exchange module. The controller may be configured to control valves which are provided between gas tanks containing gasses which can be selectively supplied to the gas reservoir. In other words, the controller may be configured to control a partial pressure of each of the gasses in the gas reservoir provided from the gas supply. In particular, the controller may be configured to supply gasses to the gas reservoir in accordance with hyperbaric treatment schedules, such as the U.S. Navy Recompression Treatment Table 6. The controller, in combination with the gas supply, may correspond to a programmable gas supply which may provide sequential gases supporting vasodilatation (push-pull, air breaks). The controller may be further configured to control the output of the gas reservoir in order to release a certain amount of gas contained therein. In combination with the control of the gas supply to the gas reservoir, the partial pressure of each of the gasses in the gas reservoir may be controlled. Therefore, the controller may be used to establish a gas mixture in the gas reservoir having a predefined composition.

In accordance with a further embodiment, the device may include a heater for tempering of the processed blood before being infused back into the subject. In particular, the heater may be used to heat the processed blood to the body temperature of the subject by means of a heating element, e.g. a conductive heating element.

In accordance with various embodiments, the device may further include a pressure gauge for determining the pressure within the gas reservoir. The controller may be coupled to the pressure gauge and be configured to control the supply of the at least one gas to the gas reservoir based on the pressure within the gas reservoir. The controller may be also configured to control the output of the gas reservoir in order to release gas from the gas reservoir based on the pressure within the gas reservoir.

In accordance with a further embodiment, the device may further include a gas detector which is configured to determine the concentration of the gas or of at least one component of the gas mixture. The gas detector may be present in the gas reservoir, for example. By means of the gas detector, the relative content of the gasses at its installation site may be determined, i.e. the partial pressure of at least one gas. The gas detector may be provided at any other site of the system where it may be useful to determine the concentration of gasses. For example, the gas detector may be provided in the last bubble filter of the system, i.e. the bubble filter closest to the output port of the device. In the last bubble filter the composition of gas remnants being removed from the blood may be determined over time. In a successful treatment scheme, the concentration of harmful inert gasses (e.g. nitrogen or helium) should decline as they are replaced by a replacement gas, such as oxygen or air. The concentration of harmful inert gasses still being present in the blood in the final stage of the device according to various embodiments (e.g. in the last gas bubble filter) may be used as a control parameter for the execution of the gas exchange procedure carried out by the present device. That is, the gas exchange or gas removal procedure may be carried out until the concentration of at least one inert gas drops below a threshold value.

In accordance with a further embodiment, the controller may be configured to determine the rate at which the at least one gas is provided to the gas reservoir based on the amount of that gas in the corresponding gas tank. The rate at which the at least one gas is provided to the gas reservoir may be further based on an estimated time until it will be possible to treat the subject in need of treatment against decompression illness in a decompression chamber. The present device may be used immediately at the site of diving after a diver has ascended too fast and will most probably suffer decompression illness. The device may be used in particular during the time of transport of the subject to a decompression chamber. In cases in which the gas tank has a limited volume of a gas left which is provided to the gas reservoir to uphold a certain (partial) pressure in the gas chamber, the controller may determine an optimal supply rate of that gas to the gas reservoir. In this context, the optimal supply rate may be one which leads to a high enough concentration of the gas in the gas reservoir during the time until the subject can be treated in a decompression chamber. In the simplest scenario the controller may be configured to determine the optimal supply rate of the at least one gas to the gas reservoir such that the remaining volume of that gas in the gas tank is evenly distributed over the time span until the subject will most probably be treated in a decompression chamber.

The present device may be used immediately after an event which will most probably lead to symptoms of decompression illness in the subject. Such an event may be a too rapid ascent of a diver. However, such an event may be also a sudden failure of a pilot's pressurization equipment at altitude which leads to the same phenomena and produces the same results. In general terms, the device may be used whenever an event has occurred in which a subject has been exposed to a sudden change of surrounding pressure and there is a risk that he or she may suffer decompression illness.

The present device may be also used in combination with a treatment of a subject in a decompression chamber. In particular, the controller may be configured to establish an atmosphere in the gas reservoir with a composition (i.e. a mixture of gases with predefined partial pressures) which corresponds to the breathing gas to be used during specified periods of the well-established hyperbaric treatment schedules. For example, if a subject is treated in a decompression chamber in accordance with US Navy Recompression Treatment Table 6, the controller may be configured to provide oxygen and air to the gas chamber in turns, as defined in Table 6. Thus, the device according to various embodiments may be used to remove at least one target gas, such as an inert gas (nitrogen, helium, argon, neon) and/or carbon dioxide, from the blood and to deposit at least one replacement gas, e.g. oxygen or a gas mixture such as air, in the blood. By providing a gas or gas mixture with a predefined composition in the gas reservoir, a chemical potential gradient across the interface between the blood compartment and the gas reservoir may be established, leading to migration of the gas molecules from the gas reservoir into the blood compartment (and consequently into the blood) or vice versa. Overall, the device according to the present invention offers a two-pronged approach for tackling depression illness. First, it is designed to efficiently remove at least one gas from the blood which, for example, has accumulated in the blood during a dive. This functionality is provided by the at least one bubble filter, the depressurizing module and the gas exchange module. In addition, the gas exchange module allows deposition of at least one gas, preferably oxygen or oxygen and an inert gas, in the blood. Preferably, as much oxygen as possible can be provided from the gas tanks to the gas exchange module as replacement gas. The oxygen concentration in the gas provided from the gas tanks to the gas reservoir may be 100% or less. If less than 100% oxygen is provided to the gas exchange reservoir, the oxygen may be provided to the gas exchange module together with an inert gas. For example, treatment schemes may be employed where pure oxygen (for example for 20 minutes) and then air (21% O2, 79%N2) are provided at intervals. Such treatment schemes may make use of a push-pull effect and have a bigger therapeutic impact compared to treatment schemes, where 100% oxygen is applied continuously.

A method for treatment and/or prophylaxis of decompression illness in a subject using the device described herein is disclosed. The method includes drawing blood from a subject by means of the input port, processing the blood in the blood processing stage of the device and infusing the processed blood back into the subject. The blood may be drawn from a vein, artery or the peritoneum and may be infused back into the subject at any one of those sites.

Further scope of applicability of the present invention will become apparent from the detailed description given hereinafter. However, it should be understood that the detailed description and specific examples, while indicating preferred embodiments of the invention, are given by way of illustration only and are not limitative of the present invention.
Fig. 1 shows a schematic view of an embodiment of the present invention.
Fig. 2 shows a more detailed view of an embodiment of the present invention.

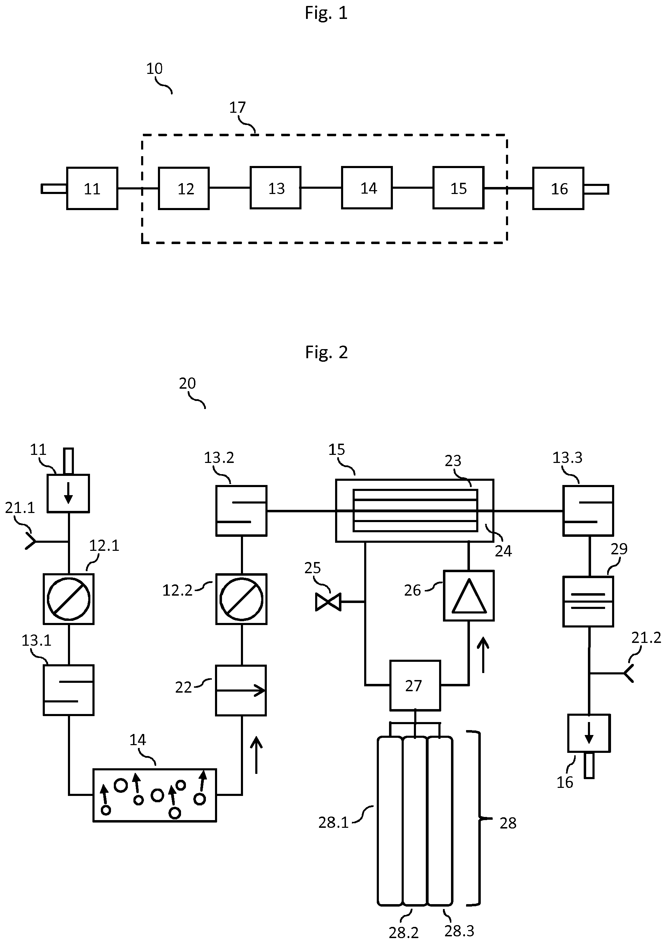

In **Fig. 1** a schematic view of an embodiment of the device 10 for treatment and/or prophylaxis of decompression illness is shown. The individual modules of the device 10, which are represented by rectangles, are (fluidically) coupled to one another by means of tubes/conduits, which are represented as connecting lines between the rectangles. The device includes an input port 11, for example a cannula, which is used to draw blood from the subject. The input port 11 is coupled to a pump 12 which promotes a flow of the drawn blood through the entire system, mainly the blood processing stage 17. The pump 12 is coupled to a bubble filter 13 which is configured to remove gas bubbles from the extracted blood. The bubble filter 13 is coupled to a depressurizing module 14 in which the blood flowing through the blood processing stage 17 is depressurized to provoke formation of gas bubbles in the blood and their subsequent release from the blood. In a sense, the depressurizing module 14 may be seen to simulate a decrease of pressure acting upon the drawn blood, thereby provoking formation of gas bubbles which are thereby released from the blood. The mechanism used in the depressurizing module 14 is substantially the same as the mechanism causing depression illness. The depressurizing module 14 is coupled to the gas exchange module 15. The gas exchange module 15 includes a gas reservoir and is configured to provide gas exchange between the gas reservoir and the blood flowing through the gas exchange module. The gas exchange module 15 is coupled to the output port 16 through which the blood processed in the blood processing stage 17 may be infused back into the subject.

The modules 11-16 comprised by the exemplary device 10 according to the invention form a closed system in which the blood is transferred from one module to the next without being exposed to the surrounding atmosphere. The modules 11-16 may be arranged in a compact fashion in a portable unit which may have the size of a suitcase and therefore may be easily transported. The device 10 may have a design which is optimized from the point of view of fluid dynamics. The blood may be transferred from one module to the next along a flow path which is free of sharp edges and/or corners at which vortices or turbulences may be formed which can ultimately lead to clumping of the blood such that a laminar flow of the blood may be maintained. In addition, the blood flow path may include an inner surface which is optimized in that it supports laminar flow. The inner surface of the flow path of the blood may have a very smooth surface which prevents formation of nucleation sites for bubbles.

The pump 12 enables usage of the system in high pressure environments, such as a decompression chamber. The pump 12 may uphold blood flow through the device 10 even when the surrounding pressure lies, for example, at 11 bar in order to simulate a dive at a depth of approximately 100 meters below the surface.

In **Fig. 2** a further embodiment 20 of the device for treatment and/or prophylaxis of decompression illness is shown. The device 20 includes all the modules from Fig. 1 which are labeled with the same reference numbers. Due to the presence of multiple instances of some devices, a suffix delimited from the respective reference number is used in Fig. 2 to denote further instances of the same device. With reference to the pump which carries the reference number 12, reference number 12.1 denotes the first pump and reference number 12.2 denotes the second pump. The modules 11-16 which have already been described in the context of Fig. 1 will not be described again in the context of Fig. 2, unless more and/or further details are to be provided.

As in the embodiment shown in Fig. 1, the exemplary embodiment 20 of the inventive device includes an input port 11 which is coupled to a first pump 12.1. The first pump 12.1 is coupled to a first bubble filter 13.1 which is in turn coupled to the depressurization module 14. The depressurization module is coupled to a second pump 12.2 via a check valve 22 which prevents the blood from flowing back into the depressurization module 14 due to its internal lower pressure. The arrow on the right side of the connection between the depressurizing module 14 and the check valve 22 indicates the (only possible) flow direction of the blood through the device 20. The second pump 12.2 is coupled to a second bubble filter 13.2 and the second bubble filter 13.2 is coupled to the gas exchange module 15.

The gas exchange module 15 includes a membrane 23 which is arranged between a gas reservoir 24 and the blood compartment of the gas exchange module 15 through which the blood is passing. The gas reservoir 24 is coupled to a gas supply 28 comprising a first gas tank 28.1, a second gas tank 28.2 and a third gas tank 28.3, each comprising a gas. The gas supply may include more or less gas tanks. One of the tanks may include oxygen and a further tank may include helium, for example. A controller 27 is interposed between the gas supply 28 and the gas reservoir 24. Each of the gas tanks 28.1-28.3 comprises a valve (not shown in the figure) which may be controlled by the controller 27 such that predetermined amounts of gas from the gas supply 28 may be supplied to the gas reservoir 24 of the gas exchange module 15. In other words, the controller 27 is configured to maintain the gas reservoir 24 at a predetermined pressure and filled with a predetermined mixture of gasses from the gas supply 28. A stage ambient 26 is provided in the gas flow path between the controller 27 and the gas reservoir 24; that flow path may be seen as an input of the gas reservoir 24. The stage ambient 26 is provided to adjust the pressure within the device (expect the depressurizing module where the pressure is always lower than the pressure within the remaining portion of the present device) to the ambient pressure, i.e. the pressure surrounding the device. The stage ambient is particularly useful when the device according to various embodiments is placed and used in a hyperbaric chamber. The arrow on the right side of the connection between the controller 27 and the stage ambient 26 indicates the direction of gas flow from the gas supply 28 to the gas reservoir 24. The gas reservoir 24 further comprises an output which is coupled to the controller 27. A venting valve 25 is coupled to the output of the gas reservoir 24 and may be controlled by the controller 27. The venting valve 25 may be used in order to release gas from the gas reservoir 24 in order to lower its pressure.

The output of the gas exchange module 15 is coupled to a third bubble filter 13.3. The third bubble filter 13.3 is coupled to a heater 29 which may be used to temper the processed blood before being infused back into the subject. For example, the processed blood may be warmed to the body temperature of the subject before being infused back into the subject. The heater may be configured as a heat exchanger. The output of the heater 29 is coupled to the output port 16 of the device 20.

As shown in Fig. 2, the device 20 further includes a first access port 21.1 and a second access port 21.2. The access ports 21.1, 21.2 may be used to sample the blood flowing through the device 20, for example to analyze its contents. The access ports 21.1, 21.2 may be also used to administer medicaments to the blood. For example, heparin and/or saline may be infused at the first access port 21.1 to prevent formation of blood clots in the device 20.

It is noted that the embodiment of device 20 for treatment and/or prophylaxis of decompression illness, as shown in Fig. 2, may be modified in various ways without departing from the scope of the invention. For example, more access ports 21.1, 21.2 may be provided and/or the access ports shown may be provided at different sites within the device 20. Also, the number and placement of pumps 12.1, 12.2 and/or bubble filters 13.1-13.3 may be different.

## Claims

1. Device (10) for treatment and/or prophylaxis of decompression illness in a subject, comprising:
an input port (11) which is configured to provide venous or peritoneal access to a subject to extract blood from the subject;
a blood processing stage (17) coupled to the input port (11) which is configured to process blood extracted from the subject; and
an output port (16) which is configured to provide venous, arterial or peritoneal access to infuse the processed blood back into the subject;
wherein the blood processing stage (17) comprises:
at least one pump (12) to promote a flow of the blood through the blood processing stage (17);
a depressurizing module (14) in which the blood flowing through the blood processing stage is depressurized to provoke formation of gas bubbles in the blood and their subsequent release from the blood;
a gas exchange module (15) comprising a gas reservoir (24) and configured to provide gas exchange between the gas reservoir and the blood flowing through gas exchange module (15);
**characterized by** at least one bubble filter (13) configured to remove gas bubbles from the extracted blood; wherein the depressurizing module (14) is a further module.

2. Device (10) of claim 1, wherein the blood flowing through the depressurizing module (14) is exposed to a pressure which is lower than the pressure in the part of the blood processing stage (17) preceding the depressurizing module (14).

3. Device (10) of claim 1 or 2, wherein in the depressurizing module (14) the blood flowing through the blood processing stage (17) is exposed to a pressure difference of 300 mbar or less between an input of the depressurizing module (14) and its interior.

4. Device (10) of any one of claims 1 to 3 wherein the gas exchange cell (15) comprises a gas supply (28) comprising oxygen and preferably at least one further gas.

5. Device (10) of any one of claims 1 to 4, wherein the at least one further gas comprises an inert gas.

6. Device (10) of any one of claims 1 to 5, wherein the gas exchange cell (15) comprises a gas permeable membrane (23) which is arranged such that its one side is in contact with the blood flowing through the gas exchange module (15) and its other side is facing the gas reservoir (24).

7. Device (10) of any one of claims 4 to 6, wherein the blood processing stage (17) further comprises a controller (27) which is configured to control the gas flow between the gas supply (28) and the gas reservoir (24).

8. Device (10) of claim 7, wherein the controller (27) is configured to control a partial pressure in the gas reservoir (24) of each of the gasses provided from the gas supply (28).

9. Device (10) of any one of claims 1 to 8, further comprising a heater (29) for tempering of the processed blood before being infused back into the subject.

10. Device (10) of any one of claims 1 to 9, further comprising a pressure gauge for determining the pressure within the gas reservoir (24).

## Patentansprüche

1. Vorrichtung (10) zur Behandlung und/oder Prophylaxe einer Dekompressionskrankheit bei einem Patienten, aufweisend:
einen Eingangsanschluss (11), der so konfiguriert ist, dass er einen venösen oder peritonealen Zugang zu einem Patienten bereitstellt, um Blut aus dem Patienten zu entnehmen;
eine Blutverarbeitungsstufe (17), die mit dem Eingangsanschluss (11) gekoppelt ist und so konfiguriert ist, dass sie das aus dem Patienten extrahierte Blut verarbeitet; und
einen Ausgangsanschluss (16), der so konfiguriert ist, dass er einen venösen, arteriellen oder peritonealen Zugang bereitstellt, um das verarbeitete Blut zurück in den Patienten zu infundieren;
wobei die Blutverarbeitungsstufe (17) aufweist:
mindestens eine Pumpe (12), um einen Fluss des Blutes durch die Blutverarbeitungsstufe (17) zu fördern;
ein Druckminderungsmodul (14), in dem das durch die Blutverarbeitungsstufe fließende Blut unter Druck gesetzt wird, um die Bildung von Gasblasen im Blut und deren anschließende Freisetzung aus dem Blut zu bewirken;
ein Gasaustauschmodul (15), das ein Gasreservoir (24) aufweist und so konfiguriert ist, dass es einen Gasaustausch zwischen dem Gasreservoir und dem durch das Gasaustauschmodul (15) fließenden Blut ermöglicht;
**gekennzeichnet durch** mindestens einen Blasenfilter (13), der so konfiguriert ist, dass er Gasblasen aus dem extrahierten Blut entfernt; wobei das Druckentlastungsmodul (14) ein weiteres Modul ist.

2. Vorrichtung (10) nach Anspruch 1, wobei das durch das Druckminderungsmodul (14) fließende Blut einem Druck ausgesetzt ist, der niedriger ist als der Druck in dem Teil der Blutverarbeitungsstufe (17), der dem Entspannungsmodul (14) vorausgeht.

3. Vorrichtung (10) nach Anspruch 1 oder 2, wobei das durch die Blutverarbeitungsstufe (17) fließende Blut in dem Druckminderungsmodul (14) einer Druckdifferenz von 300 mbar oder weniger zwischen einem Eingang des Druckminderungsmoduls (14) und seinem Inneren ausgesetzt ist.

4. Vorrichtung (10) nach einem der Ansprüche 1 bis 3, wobei die Gasaustauschzelle (15) eine Gasversorgung (28) mit Sauerstoff und vorzugsweise mindestens einem weiteren Gas aufweist.

5. Vorrichtung (10) nach einem der Ansprüche 1 bis 4, wobei das mindestens eine weitere Gas ein Inertgas ist.

6. Vorrichtung (10) nach einem der Ansprüche 1 bis 5, wobei die Gasaustauschzelle (15) eine gasdurchlässige Membran (23) aufweist, die so angeordnet ist, dass ihre eine Seite in Kontakt mit dem durch das Gasaustauschmodul (15) fließenden Blut steht und ihre andere Seite dem Gasreservoir (24) zugewandt ist.

7. Vorrichtung (10) nach einem der Ansprüche 4 bis 6, wobei die Blutverarbeitungsstufe (17) ferner ein Steuergerät (27) aufweist, das so konfiguriert ist, dass es den Gasfluss zwischen der Gasversorgung (28) und dem Gasreservoir (24) steuert.

8. Vorrichtung (10) nach Anspruch 7, wobei das Steuergerät (27) so konfiguriert ist, dass es einen Partialdruck in dem Gasreservoir (24) von jedem der von der Gasversorgung (28) bereitgestellten Gase steuert.

9. Vorrichtung (10) nach einem der Ansprüche 1 bis 8, die ferner eine Heizung (29) aufweist zum Temperieren des verarbeiteten Blutes vor der Rückinfusion in den Patienten.

10. Vorrichtung (10) nach einem der Ansprüche 1 bis 9, die ferner ein Druckmessgerät zur Bestimmung des Drucks innerhalb des Gasbehälters (24) aufweist.

## Revendications

1. Dispositif (10) pour le traitement et/ou la prophylaxie de la maladie de décompression chez un sujet, comportant:
un port d'entrée (11), qui est configuré pour fournir un accès veineux ou péritonéal à un sujet pour extraire le sang du sujet;
un stade de traitement du sang (17), couplé au port d'entrée (11), qui est configuré pour traiter du sang extrait du sujet; et
un port de sortie (16), qui est configuré pour fournir un accès veineux, artériel ou péritonéal pour réinjecter le sang traité au sujet;
dans lequel le stade de traitement du sang (17) comporte:
au moins une pompe (12) pour favoriser un écoulement du sang à travers le stade de traitement du sang (17);
un module de dépressurisation (14), dans lequel le sang traversant le stade de traitement du sang est dépressurisé pour provoquer la formation de bulles de gaz dans le sang et leur libération ultérieure du sang;
un module d'échange de gaz (15), comportant un réservoir de gaz (24), et configuré pour fournir un échange de gaz entre le réservoir de gaz (24) et le sang traversant le module d'échange de gaz (15);
**caractérisé par** au moins un filtre à bulles (13), configuré pour éliminer des bulles de gaz du sang extrait, dans lequel le module de dépressurisation (14) est un autre module.

2. Dispositif (10) selon la revendication 1, dans lequel le sang traversant le module de dépressurisation (14) est exposé à une pression, qui est inférieure à la pression dans la partie du stade de traitement du sang (17) précédant le module de dépressurisation(14).

3. Dispositif (10) selon la revendication 1 ou 2, dans lequel, dans le module de dépressurisation (14), le sang traversant le stade de traitement du sang (17) est exposé à une différence de pression de 300 mbar ou moins entre une entrée du module de dépressurisation (14) et son intérieur.

4. Dispositif (10) selon l'une quelconque des revendications 1 à 3, dans lequel la cellule d'échange de gaz (15) comporte une alimentation en gaz (28), comprenant de l'oxygène et, de préférence, au moins un autre gaz.

5. Dispositif (10) selon l'une quelconque des revendications 1 à 4, dans lequel l'au moins un autre gaz comporte un gaz inerte.

6. Dispositif (10) selon l'une quelconque des revendications 1 à 5, dans lequel la cellule d'échange de gaz (15) comporte une membrane perméable au gaz (23), qui est agencée de sorte que l'un de ses côtés est en contact avec le sang traversant le module d'échange de gaz (15), et que son autre côté est tourné vers le réservoir de gaz (24).

7. Dispositif (10) selon l'une quelconque des revendications 4 à 6, dans lequel le stade de traitement du sang (17) comporte en outre un contrôleur (27), qui est configuré pour contrôler le flux de gaz entre l'alimentation en gaz (28) et le réservoir de gaz (24).

8. Dispositif (10) selon la revendication 7, dans lequel le contrôleur (27) est configuré pour contrôler une pression partielle dans le réservoir de gaz (24) de chacun des gaz fournis à partir de l'alimentation en gaz (28).

9. Dispositif (10) selon l'une quelconque des revendications 1 à 8, comportant en outre un dispositif de chauffage (29) pour tempérer le sang traité avant de le réinjecter dans le sujet.

10. Dispositif (10) selon l'une quelconque des revendications 1 à 8, comportant en outre un manomètre pour déterminer la pression dans le réservoir de gaz (24).
